# EUROPEAN PATENT APPLICATION

(11) **EP 3 181 114 A1**
(43) Date of publication of application: **21.06.2017**
(21) Application number: 16204428.3
(22) Date of filing: 15.12.2016
(51) Int. Cl.: A61K 8/36, A61K 8/39, A61K 8/46, A61Q 19/10, A61K 8/02, A61K 8/20, C11D 17/00

(54) **CLEANSING BARS CONTAINING POLYGLYCEROL ESTERS**

(30) Priority: 16.12.2015 US 201514970783
(71) Applicant: JOHNSON & JOHNSON CONSUMER INC., Skillman, NJ 08558 (US)
(72) Inventor: DAI, Congyun, Minhang District, Shanghai 200245 (CN); FANG, Peifei, Minhang District, Shanghai 200245 (CN); FEVOLA, Michael J., Skillman, NJ New Jersey 08558 (US); FUETTERER, Tobias J., Skillman, NJ New Jersey 08558 (US)
(74) Representative: Carpmaels & Ransford LLP

(57) **Abstract**

The present invention provides a cleansing bar having a pH of about 8 or less and that contains a non-soap anionic surfactant, a polyglycerol ester having a peak melting point of about 40°C or less, a hydrophobic binder, and a water-soluble bar hardener.

## Description

### FIELD OF INVENTION

The present invention relates to cleansing bars comprising polyglycerol esters and, in particular, high foaming, mild cleansing bars comprising polyglycerol esters having a peak melting point of about 40°C or less.

### BACKGROUND OF THE INVENTION

Cleansing bars are well-known for providing a cost-effective and convenient means for washing the skin. Typical cleansing bars include soap and/or synthetic surfactants and various other ingredients to provide functional and aesthetically appealing cleansing experience.

One approach for providing cleansing bars with good lathering is exemplified in US 5,372,751 (Rys-Cicciari et al.) and relates to the use of particular combinations of surfactants. Applicants have however recognized the need for entirely new cleansing bar compositions that provide more enhancement of foaming while maintaining reduced irritation, and/or ease of manufacture.

### SUMMARY OF THE INVENTION

The present invention provides a cleansing bar that comprises a non-soap anionic surfactant, a polyglycerol ester having a peak melting point of about 40°C or less, a hydrophobic binder, and a water-soluble bar hardener. The cleansing bar has a pH of about 8 or less.

### BRIEF DESCRIPTION OF THE DRAWINGS

**Figure 1** is a graphical depiction of a complete Heat-Cool-Heat heat flow curve measured by Differential Scanning Calorimetry of a polyglycerol ester.
**Figure 2** is a graphical depiction of an expended section of a heat flow curve of a polyglycerol ester of the present invention showing the peak analysis and peak melting point temperature determination.
**Figure 3** is a graphical depiction of the results of the Cleansing Bar Foam Test for certain compositions of the present invention and comparable compositions.

### DESCRIPTION OF PREFERRED EMBODIMENTS

We have discovered that the cleansing bars of the invention exhibit a unique and unexpected combination of properties, including high lathering characteristics, relatively low irritation and ease of manufacturability. This makes the cleansing bars of this invention highly desirable for use in cosmetic or cleansing compositions for use in skin care, including baby and infant skin. The cleansing bars of this invention comprise a non-soap anionic surfactant, a hydrophobic binder, and a water-soluble bar hardener. The cleansing bars further comprise a polyglycerol ester having a peak melting point of about 40°C or less and have a pH less of about 8 or less. Surprisingly, incorporation of the polyglycerol ester into these relatively low pH cleansing bars results in a mild cleansing bar composition that is easily processed and provides better lathering than previously thought would be possible.

All percentages listed in this specification are percentages by weight, unless otherwise specifically mentioned.

Where applicable, chemicals are specified according to their INCI (International Nomenclature of Cosmetic Ingredients) Name. Additional information, including suppliers and trade names, can be found in the following which are herein incorporated by reference: the INCI monographs in the International Cosmetic Ingredient Dictionary and Handbook, 14th Edition published by the Personal Care Products Council, Washington DC and M. Friedman, and in Chemistry, Formulation, and Performance of Syndet and Combo Bars, Chapter 5 in Soap Manufacturing Technology, L. Spitz, ed., AOCS Press: Urbana, IL, 2009, pp 153-189.

As used herein, the term "cleansing bar" refers to a cleansing composition in the form of a bar, i.e., a solid (maintaining its shape rather than taking the shape of its container) under ambient conditions. The bar may be of varying shapes and cross-sections, e.g., circular, oval, square, rectangular; flat or rounded; or non-conventional shapes. Desirably, the bar is suitable to hold in one's hand(s). As such, the cleansing bar may have dimensions such that the length or longest dimension is from about 4cm to about 12cm, preferably from about 5cm to about 10cm; the width is from about 3cm to about 8cm, preferably from about 4cm to about 7cm; and a thickness from about 0.25cm to about 4 cm, preferably from about 0.5cm to about 3cm.

Upon being wet, cleansing bars release or exude a cleansing composition that has the ability to remove dirt, oils, excess sebum and the like from the skin surface and which produce a foam (i.e., a frothy mass of fine bubbles formed in or on the surface of a liquid or from a liquid). The cleansing bar is typically wet with water and applied to the skin. Rubbing the cleansing bar with one's fingers or hands, a wash cloth, or other implement, e.g. a pouf, may result in sudsing or foaming of the cleansing composition to produce a lather. The composition is then rinsed off with water.

Cleansing bars of the present invention may be used in typical personal care cleansing on adult or infant skin that is intact, or skin that has, for example, a wound or perturbed barrier. Various parts of the body may be cleansed, for example, face, body, hair, internal or external vaginal area, and the like.

### Non-Soap Anionic Surfactant

As used herein "anionic surfactant" refers to an amphiphilic molecule comprising a hydrophobic group and one or more hydrophilic groups comprising a negatively charged moiety or a moiety capable of bearing a negative charge (in the latter case, for example, as a function of acid-base properties and solution pH). As used herein "non-soap anionic surfactant" refers to an anionic surfactant other than the following: alkali (e.g. Na⁺ and K⁺), alkaline earth (e.g. Mg²⁺ and Ca²⁺), ammonium, or triethanolamine salts of saturated and unsaturated C₆-C₂₄ fatty acids, i.e. alkyl monocarboxylate salts. As one skilled in the art will recognize, soaps are most often derived from the saponification of triglycerides.

Examples of suitable non-soap anionic surfactants include the following:
- Acyl isethionates 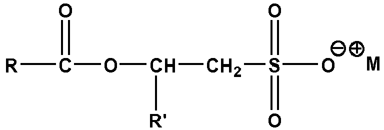 where RCO = C₈ - C₂₀ acyl (linear or branched, saturated or unsaturated) or mixtures thereof, R' = H or CH₃, M⁺ = monovalent cation, such as Sodium Cocoyl Isethionate (RCO = coco acyl, R' = H, M⁺ = Na⁺) and Sodium Lauroyl Methyl Isethionate (RCO = lauroyl, R' = CH₃, M⁺ = Na⁺);
- Alkyl sulfosuccinates 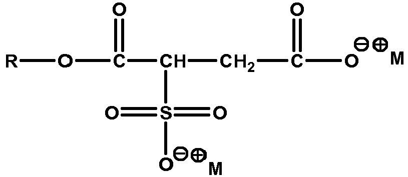 where R = C₈ - C₂₀ alkyl (linear or branched, saturated or unsaturated) or mixtures thereof and M⁺ = monovalent cation, such as Disodium Lauryl Sulfosuccinate (R = lauryl, M⁺ = Na⁺);
- α-Sulfo fatty acid esters 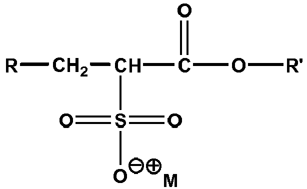 where R = C₆ - C₁₆ alkyl (linear or branched, saturated or unsaturated) or mixtures thereof, R' = C₁ - C₂ alkyl, and M⁺ = monovalent cation, such as Sodium Methyl 2-Sulfolaurate (R = C₁₀H₂₁, R' = methyl, CH₃, and M⁺ = Na⁺);
- α -Sulfo fatty acid salts 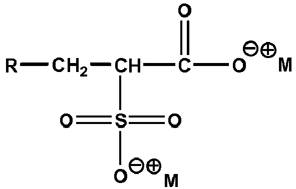 where R = C₆ - C₁₆ alkyl (linear or branched, saturated or unsaturated) or mixtures thereof, M⁺ = monovalent cation, such as Disodium 2-Sulfolaurate (R = C₁₀H₂₁, M⁺ = Na⁺);
- Alkyl sulfoacetates 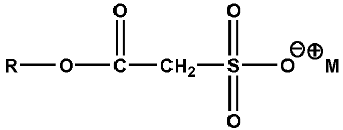 where R = C₆ - C₁₈ alkyl (linear or branched, saturated or unsaturated) or mixtures thereof, M⁺ = monovalent cation, such as Sodium Lauryl Sulfoacetate (R = lauryl, C₁₂H₂₅, M⁺ = Na⁺);
- Alkyl sulfates 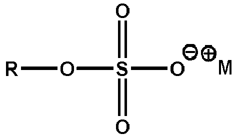 where R = C₈ - C₂₀ alkyl (linear or branched, saturated or unsaturated) or mixtures thereof. Specific examples include TEA-Lauryl Sulfate (R = lauryl, C₁₂H₂₅, M⁺ = ⁺HN(CH₂CH₂OH)₃), Sodium Lauryl Sulfate (R = lauryl, C₁₂H₂₅, M⁺ = Na⁺), and Sodium Coco-Sulfate (R = coco alkyl, M⁺ = Na⁺);
- Alkyl glyceryl ether sulfonates or alkoxyl hydroxypropyl sulfonates: 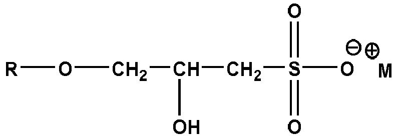 where R = C₈ - C₂₄ alkyl (linear or branched, saturated or unsaturated) or mixtures thereof and M⁺ = monovalent cation, such as Sodium Cocoglyceryl Ether Sulfonate (R = coco alkyl, M⁺ = Na⁺);
- α-olefin sulfonates prepared by sulfonation of long chain alpha olefins. Alpha olefin sulfonates consist of mixtures of alkene sulfonates, 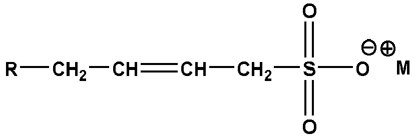 where R = C₄ - C₁₈ alkyl or mixtures thereof and M⁺ = monovalent cation, and hydroxyalkyl sulfonates, 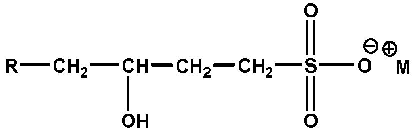 where R = C₄ - C₁₈ alkyl or mixtures thereof and M⁺ = monovalent cation. Examples include Sodium C12-14 Olefin Sulfonate (R = C₈ - C₁₀ alkyl, M⁺ = Na⁺) and Sodium C14-16 Olefin Sulfonate (R = C₁₀ - C₁₂ alkyl, M⁺ = Na⁺);
- Alkyl sulfonates or paraffin sulfonates: 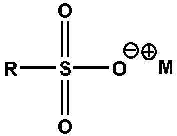 where R = C₈ - C₂₄ alkyl (linear or branched, saturated or unsaturated) or mixtures thereof and M⁺ = monovalent cation. Examples include Sodium C13-17 Alkane Sulfonate (R = C₁₃ - C₁₇ alkyl, M⁺ = Na⁺) and Sodium C14-17 Alkyl Sec Sulfonate (R = C₁₄ - C₁₇ alkyl, M⁺ = Na⁺);
- Alkylaryl sulfonates or linear alkyl benzene sulfonates 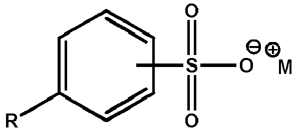 where R = C₆ - C₁₈ alkyl (linear, saturated or unsaturated) or mixtures thereof and M⁺ = monovalent cation. Examples include Sodium Deceylbenzenesulfonate (R = C₁₀ alkyl, M⁺ = Na⁺) and Ammonium Dodecylbenzensulfonate (R = C₁₂ alkyl, M⁺ = NH₄⁺);
- Alkyl ether sulfates 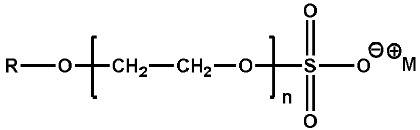 where R = C₈ - C₂₄ alkyl (linear or branched, saturated or unsaturated) or mixtures thereof, n = 1 - 12, and M⁺ = monovalent cation. Examples include Sodium Laureth Sulfate (R = C₁₂ alkyl, M⁺ = Na⁺, n = 1 - 3), Ammonium Laureth Sulfate (R = C₁₂ alkyl, M⁺ = NH₄⁺, n = 1 - 3), and Sodium Trideceth Sulfate (R = C₁₃ alkyl, M⁺ = Na⁺, n = 1 - 4);
- Alkyl monoglyceride sulfates 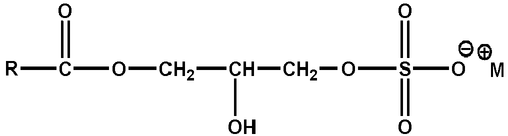 where RCO = C₈ - C₂₄ acyl (linear or branched, saturated or unsaturated) or mixtures thereof and M⁺ = monovalent cation. Examples include Sodium Cocomonoglyceride Sulfate (RCO = coco acyl, M⁺ = Na⁺) and Ammonium Cocomonoglyceride Sulfate (RCO = coco acyl, M⁺ = NH₄⁺);
- Alkyl ether carboxylates 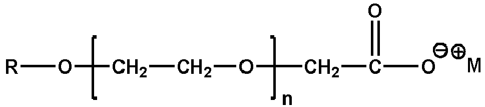 where R = C₈ - C₂₄ alkyl (linear or branched, saturated or unsaturated) or mixtures thereof, n = 1 - 20, and M⁺ = monovalent cation. Examples include Sodium Laureth-13 Carboxylate (R = C₁₂ alkyl, M⁺ = Na⁺, n = 13), and Sodium Laureth-3 Carboxylate (R = C₁₂ alkyl, M⁺ = Na⁺, n = 3);
- Alkyl ether sulfosuccinates 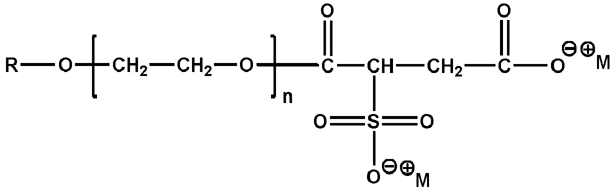 where R = C₈ - C₂₀ alkyl (linear or branched, saturated or unsaturated) or mixtures thereof, n = 1 - 12, and M⁺ = monovalent cation, such as Disodium Laureth Sulfosuccinate (R = lauryl, n = 1 - 4, and M⁺ = Na⁺);
- Dialkyl sulfosuccinates 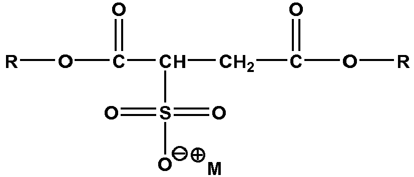 where R = C₆ - C₂₀ alkyl (linear or branched, saturated or unsaturated) or mixtures thereof and M⁺ = monovalent cation, such as Diethylhexyl Sodium Sulfosuccinate (R = 2-ethylhexyl, M⁺ = Na⁺);
- Alkylamidoalkyl sulfosuccinates 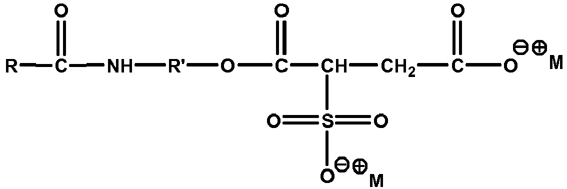 where R = C₈ - C₂₀ alkyl (linear or branched, saturated or unsaturated) or mixtures thereof, R' = C₂ - C₄ alkyl (linear or branched), and M⁺ = monovalent cation, such as Disodium Cocamido MIPA-Sulfosuccinate (RCO = coco acyl, R' = isopropyl, M⁺ = Na⁺);
- Alkyl sulfosuccinamates 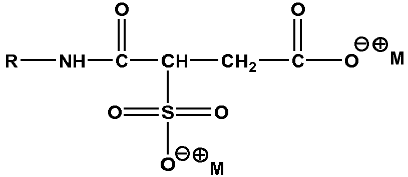 where R = C₈ - C₂₀ alkyl (linear or branched, saturated or unsaturated) or mixtures thereof and M⁺ = monovalent cation, such as Disodium Stearyl Sulfosuccinamate (R = stearyl, C₁₈H₃₇, M⁺ = Na⁺);
- Acyl glutamates 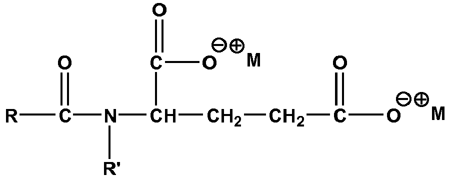 where RCO = C₆ - C₂₀ acyl (linear or branched, saturated or unsaturated) or mixtures thereof, R' = H or CH₃, M⁺ = monovalent cation, such as Disodium Cocoyl Glutamate (RCO = coco acyl, R' = H, M⁺ = Na⁺) and Disodium Lauroyl Glutamate (RCO = lauroyl, R' = H, M⁺ = Na⁺);
- Acyl aspartates 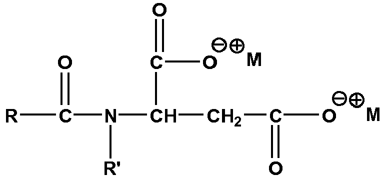 where RCO = C₆ - C₂₀ acyl (linear or branched, saturated or unsaturated) or mixtures thereof, R' = H or CH₃, M⁺ = monovalent cation, such as Disodium N-Lauroyl Aspartate (RCO = lauroyl, R' = H, M⁺ = Na⁺);
- Acyl taurates 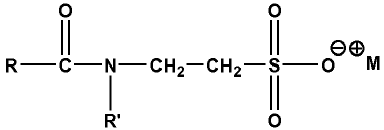 where RCO = C₆ - C₂₀ acyl (linear or branched, saturated or unsaturated) or mixtures thereof, R' = H or CH₃, M⁺ = monovalent cation, such as Sodium Methyl Cocoyl Taurate (RCO = coco acyl, R' = CH₃, M⁺ = Na⁺) and Sodium Cocoyl Taurate (RCO = lauroyl, R' = H, M⁺ = Na⁺);
- Acyllactylates 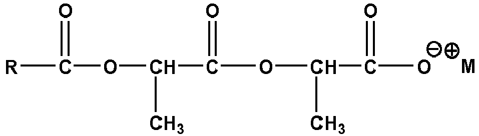 where RCO = C₈ - C₂₀ acyl (linear or branched, saturated or unsaturated) or mixtures thereof, M⁺ = monovalent cation, such as Sodium Lauroyl Lactylate (RCO = lauroyl, M⁺ = Na⁺);
- Acyl glycinates and acyl sarcosinates 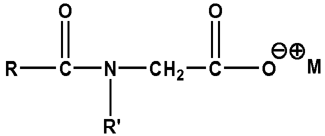 where RCO = C₈ - C₂₀ acyl (linear or branched, saturated or unsaturated) or mixtures thereof, R' = H (glycinate) or CH₃ (sarcosinate), M⁺ = monovalent cation, such as Sodium Cocoyl Glycinate (RCO = coco acyl, R' = H, M⁺ = Na⁺), Ammonium Cocoyl Sarcosinate (RCO = coco acyl, R' = CH₃, M⁺ = NH₄⁺) and Sodium Lauroyl Sarcosinate (RCO = lauroyl, R' = CH₃, M⁺ = Na⁺);
- Anionic derivatives of alkyl polyglucosides (APGs), including: Sodium Lauryl Glucoside Carboxylate, Disodium Coco-Glucoside Citrate, Sodium Coco-Glucoside Tartrate, Disodium Coco-Glucoside Sulfosuccinate; Sodium Cocoglucosides Hydroxypropylsulfonate, Sodium Decylglucosides Hydroxypropylsulfonate, Sodium Laurylglucosides Hydroxypropylsulfonate; Sodium Hydroxypropylsulfonate Cocoglucoside Crosspolymer, Sodium Hydroxypropylsulfonate Decylglucoside Crosspolymer, Sodium Hydroxypropylsulfonate Laurylglucoside Crosspolymer; Anionic polymeric APG derivatives, such as those described in O'Lenick, U.S. Pat. Nos. 7,507,399; 7,375,064; and 7,335,627); and combinations of two or more thereof, and the like.

Preferred non-soap anionic surfactants include: acyl isethionates, e.g. Sodium Cocoyl Isethionate; alkyl sulfosuccinates, e.g. Disodium Lauryl Sulfosuccinate; α-sulfo fatty acid esters, e.g. Sodium Methyl 2-Sulfolaurate; α -sulfo fatty acids, e.g. Disodium 2-Sulfolaurate; alkyl glyceryl ether sulfonates, e.g. Sodium Cocoglyceryl Ether Sulfonate; alkyl sulfates, e.g. Sodium Coco-Sulfate, and combinations of two or more thereof. Especially preferred are acyl isethionates. In certain preferred embodiments the acyl isethionate is Sodium Cocoyl Isethionate.

In certain preferred embodiments, the compositions of this invention comprise from about 30 weight percent or more to about 70 weight percent or less of total non-soap anionic surfactants, based on total weight of cleansing bar. In certain more preferred embodiments, the compositions comprise from about 35 to about 60 weight percent of total non-soap anionic surfactants, even more preferably from about 35 to about 55 weight percent, and most preferably from about 40 to about 50 weight percent total non-soap anionic surfactants.

### Polyglycerol ester

As used herein, the term "Polyglyceryl ester (PGE)" is defined as the ester formed by reacting a "polyglycerol (PG) moiety" and a "hydrophobic moiety" containing a carboxylic acid group (RCOOH), any of its salt and anhydride forms or its respective acyl halide form, like e.g. an acyl chloride.

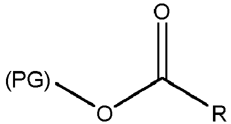

As used herein, a "polyglycerol moiety" means a linear, branched, and/or cyclic polyether moiety comprising two or more glyceryl repeat units. Polyglycerol moieties may be derived via any of a variety of synthetic routes, including but not limited to condensation polymerization of glycerol, ring-opening polymerization of glycerol carbonate, and ring-opening polymerization of glycidol. In certain embodiments, polyglycerol moieties comprise homopolyethers wherein all of the repeat units are glyceryl repeat units. In certain other embodiments, the polyglycerol moieties are copolyethers, that is, they comprise both glyceryl repeat units and additional polyether repeat units that are not glyceryl repeat units. For example, glycerol may be copolymerized with 1,3-propanediol to yield a copolyether comprising both glyceryl repeat units described above and oxypropylene repeat units.

A "hydrophobic moiety" is hereby defined as a nonpolar moiety that contains at least one of the following: (a) a carbon-carbon chain of at least four carbons in which none of the four carbons is a carbonyl carbon or has a hydrophilic moiety bonded directly to it; (b) two or more alkyl siloxy groups (-[Si(R)₂-O]-); and/or (c) two or more oxypropylene groups in sequence. A hydrophobic moiety may be, or include, linear, cyclic, aromatic, saturated or unsaturated groups. In certain preferred embodiments, hydrophobic moieties comprise a carbon chain of at least six or more carbons, more preferably seven or more carbons in which none of the carbons in such chain have a hydrophilic moiety bonded directly thereto. Certain other preferred hydrophobic moieties include moieties comprising a carbon chain of about eight or more carbon atoms, more preferably about 10 or more carbon atoms in which none of the carbons in such chain have a hydrophilic moiety bonded directly thereto. Examples of hydrophobic functional moieties may include esters, ketones, amides, carbonates, urethanes, carbamates, or xanthate functionalities, and the like, having incorporated therein or attached thereto a carbon chain of at least four carbons in which none of the four carbons has a hydrophilic moiety bonded directly to it. Other examples of hydrophobic moieties include groups such as poly(oxypropylene), poly(oxybutylene), poly(dimethylsiloxane), fluorinated hydrocarbon groups containing a carbon chain of at least four carbons in which none of the four carbons has a hydrophilic moiety bonded directly to it, and the like.

Examples of PGEs include polyglyceryl-10 laurate where PG = polyglyceryl moiety comprising ten (10) glyceryl repeat units, and R = C11H23, as well as, polyglyceryl-10 caprylate/caprate, polyglyceryl-10 cocoate, polyglyceryl-10 myristate, polyglyceryl-10 palmitate, polyglyceryl-10 oleate, polyglyceryl-12 laurate, and the like. PGEs of the present invention may include polyglyceryl moieties bearing multiple ester substitutions (i.e. the PGEs may be monoesters, diesters, triesters, etc.).

As used herein, "PGE having a peak melting point of about 40°C or less (PGE_{≤40})" means a PGE having a phase transition from a solid-like state into a liquid-like state at or below about 40°C. "Liquid-like" refers to a state for which a viscosity can be measured using a rheometer with e.g. a cone-plate or parallel plate geometry as detailed in this specification. "Solid-like" refers to a state which allows having the PGE in powder- or pastille-, or e.g. flake-form. The phase transition temperature is to be determined by Differential Scanning Calorimetry (DSC) as detailed in this specification. Examples of PGEs having a peak melting point of about 40°C or less include, but are not limited to: polyglyceryl-4 caprylate/caprate, polyglyceryl-5 caprylate/caprate, polyglyceryl-6 caprylate/caprate, polyglyceryl-7 caprylate/caprate, polyglyceryl-8 caprylate/caprate, polyglyceryl-9 caprylate/caprate, polyglyceryl-10 caprylate/caprate, polyglyceryl-4 caprate, polyglyceryl-5 caprate, polyglyceryl-6 caprate, polyglyceryl-7 caprate, polyglyceryl-8 caprate, polyglyceryl-9 caprate, polyglyceryl-10 caprate, polyglyceryl-4 laurate, polyglyceryl-5 laurate, polyglyceryl-6 laurate, polyglyceryl-7 laurate, polyglyceryl-8 laurate, polyglyceryl-9 laurate, polyglyceryl-10 laurate, polyglyceryl-6 cocoate, polyglyceryl-7 cocoate, polyglyceryl-8 cocoate, polyglyceryl-9 cocoate, polyglyceryl-10 cocoate, polyglyceryl-11 cocoate, polyglyceryl-12 cocoate, polyglyceryl-6 myristate, polyglyceryl-7 myristate, polyglyceryl-8 myristate, polyglyceryl-9 myristate, polyglyceryl-10 myristate, polyglyceryl-11 myristate, polyglyceryl-12 myristate, polyglyceryl-10 oleate, polyglyceryl-11 oleate, polyglyceryl-12 oleate, and combinations of two or more thereof.

To the surprise of the applicants, cleansing bars as described herein containing PGE_{≤40} did show significantly improved foaming compared to respective bars without the PGE_{≤40} or with a PGE having a peak melting point greater than about 40 C. Cleansing bars of the present invention also provide a soft smooth skin feel and maintain their mildness.

According to certain embodiments, the number of repeat units in the PG moiety of the PGE_{≤40} is from about 2 to 20, more preferably from about 2 to 12, and more preferably from about 4 to 10. The number of PG repeat units typically is determined via hydroxyl group determination as taught in US 8,961,945, the content of which is hereby incorporated herein in its entirety.

According to certain embodiments, the PGE_{≤40} is used in a concentration from about 0.1% or greater to about 25% by weight in the composition, more preferably from about 0.1 % or greater to about 10% by weight, even more preferably from about 0.1 % or greater to about 7% by weight, and even more preferable from about 0.1 % or greater to about 5% by weight in the composition. Preferably, the PGE_{≤40} is in a concentration from about 1% to about 10%, more preferably from about 1% to about 7.5%, even more preferably from about 2% to about 6% in the composition.

To obtain good processability and usage properties, the composition is stabilized with a hydrophobic binder that functions as a binder and as a plasticizer to facilitate better extrusion and stamping of the bar. As used herein "hydrophobic binder" refers to a compound that includes a hydrophobic moiety and is generally insoluble in water. The hydrophobic binder is preferably solid at room temperature but either melts or becomes malleable and/or flowable at room and elevated temperatures (≥ 30 °C). The hydrophobic binder may also have additional functions, such as an emollient to the skin being cleansed.

Examples of classes of suitable hydrophobic binders include fatty acids, fatty alcohols, esters of alcohols with fatty acids, polyol esters, waxes, mixed glycerides, triglycerides, hydrogenated tri glycerides, hydrogenated metathesis products of unsaturated triglycerides, and combinations thereof.

Suitable fatty acids and fatty alcohols include those having from about 8 to about 24 carbon atoms, such as those having at least 16 carbon atoms, for example stearic acid and steryl alcohol. Suitable esters of alcohols with fatty acids include those having at least about 16 carbon atoms, for example synthetic beeswax. Suitable polyol esters include glyceryl esters such as Glyceryl Stearate or Glyceryl Distearate; sorbitan esters, such as Sorbitan Sesqustearate or Sorbitan Tristearate, and methyl glucoside esters, such as Methyl Glucose Dioleate or Methyl Glucose Distearate.

As used herein "wax" refers to hydrophobic compounds having a melting point that is above 30°C. The wax may be hydrocarbon, animal, vegetable, mineral or synthetic. According to certain embodiments the wax includes, or is selected from the group consisting of, straight or branched chain alkanes or alkenes, ketones, diketones, primary or secondary alcohols, aldehydes, sterol esters, terpenes, and esters, such as those having a carbon chain length ranging from C₁₂-C₃₈. According to certain preferred embodiments, the wax includes esters of alcohol (glycerol or other than glycerol) and long chain fatty acids. Suitable naturally occurring waxes include Beeswax, Lanolin Wax, Copernicia Cerifera (Carnauba) Wax, and Simmondsia Chinensis (Jojoba) Seed Wax. Suitable petroleum derived waxes include Paraffin, Microcrystalline Wax, and Petrolatum. Suitable mixed glycerides include those having an average carbon chain length at least about 12, for example, Cocoglycerides, Olive Glycerides, Palm Glycerides, and Palm Kernal Glycerides. Suitable triglycerides, include Butyrospermum Parkii (Shea) Butter, Theobroma Cacao (Cocoa) Seed Butter, Simmondsia Chinensis (Jojoba) Seed Oil, and Cocos Nucifera (Coconut) Oil. Suitable hydrogenated triglycerides, include Hydrogenated C12-18 Triglycerides, Hydrogenated Castor Oil, and Hydrogenated Jojoba Oil. Suitable Hydrogenated metathesis products of unsaturated triglycerides, include Hydrogenated Soy Polyglycerides.

Any suitable total amount of hydrophobic binder may be used in cleansing bars of the present invention. In certain embodiments, the total concentration of hydrophobic binder is from 5 percent to about 50 percent. In certain preferred embodiments, the total concentration of hydrophobic binder is from 10 percent to about 50 percent, preferably from about 15 percent to about 50 percent, more preferably from 20 percent to about 50 percent, and even more preferably 20 percent to about 40 percent.

According to certain preferred embodiments, the hydrophobic binder includes a C₈ - C₂₄ fatty acid of the formula R-COOH, where R = C₇ - C₂₃ alkyl, linear or branched, saturated or unsaturated fatty acid. According to certain particularly preferred embodiments the fatty acid includes or consists of a majority of stearic acid, palmitic acid, blends of C₁₆ - C₁₈ linear saturated fatty acids, coconut fatty acids, or combinations thereof

As used herein "water soluble bar hardener" refers to a water-soluble material that tends to provide increased hardness to the cleansing bar. Examples of classes of suitable water-soluble bar hardeners include inorganic metal cation salts of organic or inorganic acids. Examples of inorganic metal cation salts of organic acids include, for example, (sodium) salts isethionic acid, lactic acid, and citric acid. Examples of inorganic metal cation salts of inorganic acids include, for example, simple sodium salts, such as sodium chloride and sodium sulfate. According to certain preferred embodiments, the water soluble bar hardener is selected from sodium chloride and sodium isethionate.

Any suitable total amount of water soluble bar hardener may be used in cleansing bars of the present invention. In certain embodiments, the total concentration of water soluble bar hardener is from 0.25 percent to about 10 percent. In certain preferred embodiments, the total concentration of water soluble bar hardener is from 0.5 percent to about 5 percent, preferably from about 0.5 percent to about 3 percent.

Cleansing bars of the present invention may further include a zwitterionic surfactant. As used herein, "zwitterionic surfactant" refers to as used herein refers to an amphiphilic molecule comprising a hydrophobic group and one or more hydrophilic groups comprising two moieties of opposite formal charges or capable of bearing opposite formal charges as a function of acid-base properties and solution pH. Any suitable zwitteronic surfactant may be used in the present invention.

Examples of suitable zwitteronic surfactants include:
- Alkyl betaines of the formula: 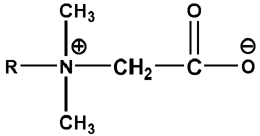 where R = C₆ - C₂₄ alkyl (saturated or unsaturated) or mixtures thereof. Examples include Coco-Betaine (R = coco alkyl), Lauryl Betaine (R = lauryl, C₁₂H₂₅), and Oleyl Betaine (R = oleyl, C₁₈H₃₅).
- Alkyl hydroxysultaines of the formula: 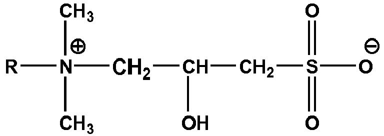 where R = C₆ - C₂₄ alkyl (saturated or unsaturated) or mixture thereof. Examples include Coco-Hydroxysultaine (R = coco alkyl) and Lauryl Hydroxysultaine (R = lauryl, C₁₂H₂₅).
- Alkyl sultaines of the formula: 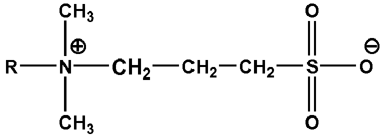 where R = C₆ - C₂₄ alkyl (saturated or unsaturated) or mixture thereof. Examples include Lauryl Sultaine (R = lauryl, C₁₂H₂₅) and Coco-Sultaine (R = coco alkyl).
- Alkylamidoalkyl betaines of the formula: 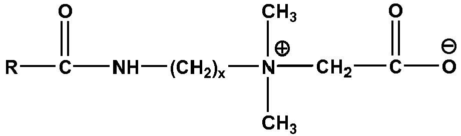 where RCO = C₆ - C₂₄ acyl (saturated or unsaturated) or mixtures thereof and *x* = 1 - Examples include Cocamidoethyl Betaine (RCO = coco acyl, *x* = 2), Cocamidopropyl Betaine (RCO = coco acyl, *x* = 3), Lauramidopropyl Betaine (RCO = lauroyl, and *x* = 3), Myristamidopropyl Betaine (RCO = myristoyl, and *x* = 3), Soyamidopropyl Betaine (R = soy acyl, *x* = 3), and Oleamidopropyl Betaine (RCO = oleoyl, and *x* = 3).
- Alkylamidoalkyl hydroxysultaines of the formula: 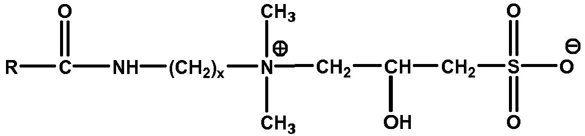 where RCO = C₆ - C₂₄ acyl (saturated or unsaturated) or mixtures thereof. Examples include Cocamidopropyl Hydroxysultaine (RCO = coco acyl, *x* = 3), Lauramidopropyl Hydroxysultaine (RCO = lauroyl, and *x* = 3), Myristamidopropyl Hydroxysultaine (RCO = myristoyl, and *x* = 3), and Oleamidopropyl Hydroxysultaine (RCO = oleoyl, and *x* = 3).
- Alkylamidoalkyl sultaines of the formula: 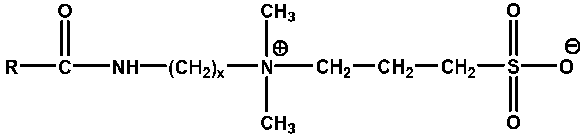 where RCO = C₆ - C₂₄ acyl (saturated or unsaturated) or mixtures thereof Examples include Cocamidopropyl Sultaine (RCO = coco acyl, *x* = 3), Lauramidopropyl Sultaine (RCO = lauroyl, and *x* = 3), Myristamidopropyl Sultaine (RCO = myristoyl, and *x* = 3), Soyamidopropyl Betaine (RCO = soy acyl, *x* = 3), and Oleamidopropyl Betaine (RCO = oleoyl, and *x* = 3).
- Alkyl phosphobetaines of the formula: 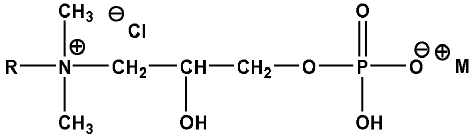 where R = C₆ - C₂₄ alkyl (saturated or unsaturated) or mixtures thereof and M⁺ = monovalent cation, such as Sodium Coco PG-Dimonium Chloride Phosphate, where R = coco alkyl and M⁺ = Na⁺.
- Phospholipids of the formula: 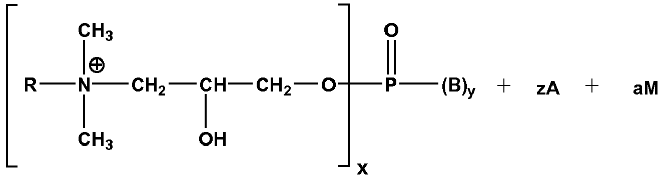 where R = C₆ - C₂₄ alkyl (saturated or unsaturated) or mixtures thereof, *x* = 1 - 3 or mixtures thereof, x + *y* = 3, *z* = *x*, *a* = 0 to 2, *B* = O⁻ or OM, A = Anion, and M = Cation (refer to U.S. Pat. Nos. 5,215,976; 5,286,719; 5,648,348; and 5,650,402), such as Sodium Coco PG-Dimonium Chloride Phosphate, where R = coco alkyl, *x* = 2, *B* = O⁻, *y* = 1, *z* = 1, A = Cl⁻*, a* = 1, and M=Na⁺.
- Phospholipids of the formula: 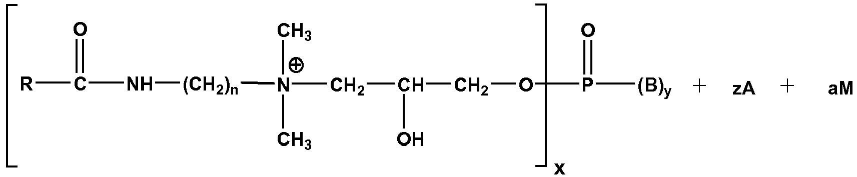 where RCO = C₆ - C₂₄ acyl (saturated or unsaturated) or mixtures thereof, n = 1 - 4, *x* = 1 - 3 or mixtures thereof, x + *y* = 3, *z* = *x, a =* 0 to 2, *B* = O⁻ or OM, A = anion, and M = cation (e.g, U.S. Pat. Nos. 5,215,976; 5,286,719; 5,648,348; and 5,650,402). Examples include Cocamidopropyl PG-Dimonium Chloride Phosphate (RCO = coco acyl, *n* = 3, *x* = 3, *z* = 3, A = Cl⁻, B and M are absent, *y* = 0, and *a* = 0) and Myristamidopropyl PG-Dimonium Chloride Phosphate (RCO = myristoyl, *n =* 3, *x* = 3, *z* = 3, A = Cl⁻, B and M are absent, *y* = 0, and *a* = 0).
- Amphoacetates of the formula: 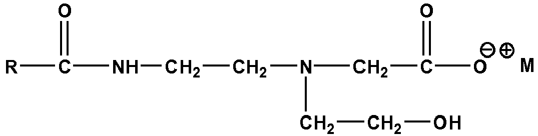 where RCO = C₆ - C₂₄ acyl (saturated or unsaturated) or mixtures thereof and M⁺ = monovalent cation. Examples include Sodium Lauroamphoacetate (RCO = lauroyl and M⁺ = Na⁺) and Sodium Cocoamphoacetate (RCO = coco acyl and M⁺ = Na⁺).
- Amphodiacetates of the formula: 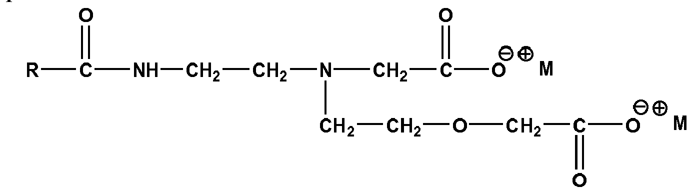 and 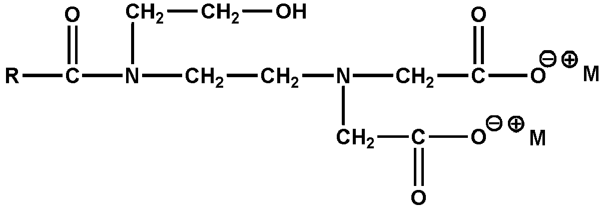 where RCO = C₆ - C₂₄ acyl (saturated or unsaturated) or mixtures thereof and M⁺ = monovalent cation. Examples include Disodium Lauroamphodiacetate (RCO = lauroyl and M = Na⁺) and Disodium Cocoamphodiacetate (RCO = coco acyl and M = Na⁺).
- Amphopropionates of the formula: 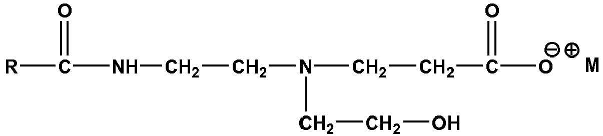 where RCO = C₆ - C₂₄ acyl (saturated or unsaturated) or mixtures thereof and M⁺ = monovalent cation. Examples include Sodium Lauroamphopropionate (RCO = lauroyl and M⁺ = Na⁺) and Sodium Cocoamphopropionate (RCO = coco acyl and M⁺ = Na⁺).
- Amphodipropionates of the formula: 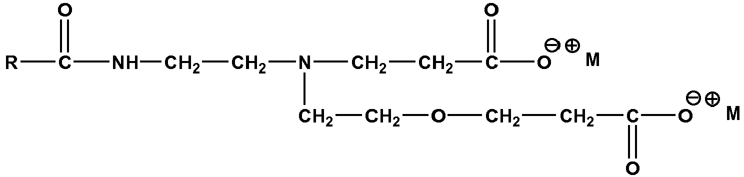 and 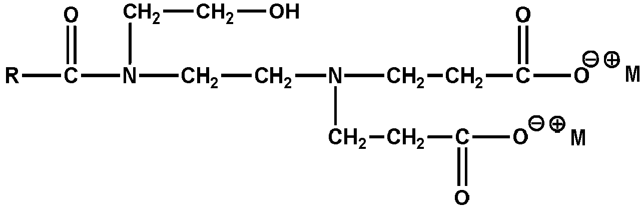 where RCO = C₆ - C₂₄ acyl (saturated or unsaturated) or mixtures thereof and M⁺ = monovalent cation. Examples include Disodium Lauroamphodipropionate (RCO = lauroyl and M⁺ = Na⁺) and Disodium Cocoamphodipropionate (RCO = coco acyl and M⁺ = Na⁺).
- Amphohydroxypropylsulfonates of the formula: 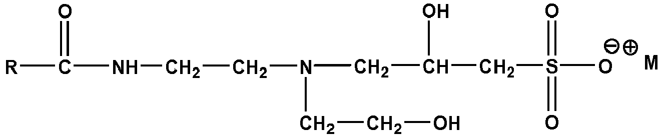 where RCO = C₆ - C₂₄ acyl (saturated or unsaturated) or mixtures thereof and M⁺ = monovalent cation, such as Sodium Lauroamphohydroxypropylsulfonate (RCO = lauroyl and M⁺ = Na⁺) and Sodium Cocoamphohydroxypropylsulfonate (RCO = coco acyl and M⁺ = Na⁺).
- Amphohydroxyalkylphosphates of the formula: 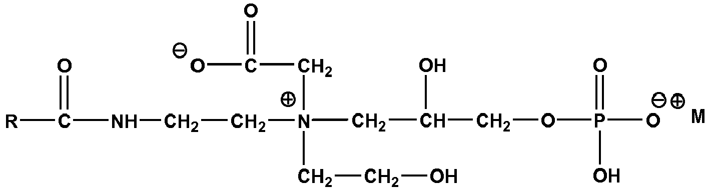 where RCO = C₆ - C₂₄ acyl (saturated or unsaturated) or mixtures thereof and M⁺ = monovalent cation, such as Sodium Lauroampho PG-Acetate Phosphate (RCO = lauroyl and M⁺ = Na⁺).
- Alkyl amine oxides of the formula: 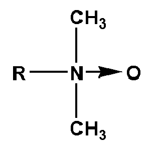 where R = C₆ - C₂₄ alkyl (saturated or unsaturated) or mixtures thereof Examples include Cocamine Oxide (R = coco alkyl) and Lauramine Oxide (RCO = lauryl).
- Alkylamidoalkyl amine oxides of the formula: 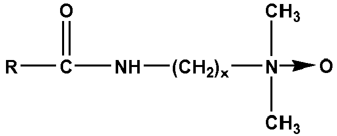 where RCO = C₆ - C₂₄ acyl (saturated or unsaturated) or mixtures thereof and *x* = 1 - Examples include Cocamidopropylamine Oxide (RCO = coco acyl, *x* = 3) and Lauramidopropylamine Oxide (RCO = lauroyl, *x* = 3); and combinations of two or more thereof, and the like.

According to certain preferred embodiments, the zwitterionic surfactant is selected from the group consisting of alkyl betaines, alkyl hydroxysultaines, alkylamidoalkyl betaines, alkylamidoalkyl hydroxysultaines, amphohydroxypropylsulfonates, and combinations of two or more thereof.

In certain preferred embodiments, the cleansing bars of the invention comprise, from greater than about 0 to less than about 10 weight percent of total zwitterionic surfactants based on total active amount of surfactant(s) in the total weight of composition. In certain more preferred embodiments, the cleansing bars of the invention comprise from about 0.1 to about 10 weight percent of total zwitterionic surfactants. In certain even more preferred embodiments, cleansing bars of the invention have from about 0.5 to about 7.5 weight percent total zwitterionic surfactants. In more preferred embodiments, formulas have from about 0.5 to about 5 weight percent total zwitterionic surfactants. In most preferred embodiments formulas have from about 1 to about 4 weight percent total zwitterionic surfactants.

According to certain embodiments of the invention, water may be included in the cleansing bar. The water may be indirectly added as a part of other ingredients or may be intentionally added to improve bar properties. According to one embodiment, the concentration of water in the cleansing bar is from about 1 percent to about 20 percent, preferably from about 2 percent to about 15 percent, more preferably from about 3 percent to about 12 percent, even more preferably from about 4 percent to about 10 percent.

According to certain embodiments of the invention, soap may be included in the cleansing bar. As used herein, the term "soap" shall include alkali (e.g. Na⁺ and K⁺) and alkaline earth (e.g. Mg²⁺ and Ca²⁺), ammonium, or triethanolamine salts of saturated and unsaturated C₆-C₂₄ fatty acids, i.e. alkyl monocarboxylate salts. However, in order to maintain a pH of the cleansing bar that is less than about 8, it is highly desirable, according to certain embodiments, to limit the amount of soap in the cleansing bar. In certain embodiments, the concentration of soap is less than about 10 percent, preferably less than about 5 percent, more preferably less than about 1 percent, and, in certain embodiments, free of soap.

Cleansing bars of the present invention may further include additional ingredients, including those found in conventional cleansing bars. Examples of additional ingredients include but are not limited to nonionic surfactants, hydrophilic binders, humectants, conditioning agents, opacifying agents, chelating agents, conditioning agents, fillers, exfoliants, preservatives, skin benefit agents, and fragrances.

Examples of suitable nonionic surfactants include, but are not limited to Alkyl polyglycosides, and Polyhydroxy fatty acid amides. Examples of suitable Alkyl polyglycosides include Lauryl Glucoside, Coco-glucoside, and Capryl/Lauryl Wheat Bran/Straw Glycosides. Examples of suitable Polyhydroxy fatty acid amides include. Lauroyl Methyl Glucamide. The nonionic surfactant may be present in an amount of from about 0 percent to about 30 percent, such as 0 percent to about 10 percent.

Examples of suitable hydrophilic binders include Polyethylene glycols (e.g. PEG-x, where x = DP of PEG and ranges from about 10 to about 800). Ethoxylated fatty alcohols (e.g. Steareth-100), and Fatty acid ethoxylates (e.g. PEG-100 Stearate). The hydrophilic binder may be present in an amount of from about 0 percent to about 60 percent, such as 0 percent to about 20 percent.

Examples of suitable humectants include polyols such as glycerin, propylene glycol, propanediol, 1,4-Butanediol, 1,3-Butanediol, 1,2-Butanediol, Hydroxyethyl Urea, Sorbitol, Sorbitan, Xylitol and Polyglycerols (e.g. Polyglycerin-z, where z = 2 - 20). The humectant may be present in an amount of from about 0 percent to about 30 percent, such as 0 percent to about 10 percent.

Examples of suitable conditioning agents include cationic or amphoteric water-soluble polymers and proteins. Examples of suitable cationic or amphoteric water-soluble polymers include Polyquaterniums, such as Polyquaternium-7, -10, -39, or -67, Guar Hydroxypropyltrimonium Chloride, and Cassia Hydroxypropyltrimonium Chloride. Examples of suitable proteins include hydrolyzed proteins, such as Hydrolyzed Wheat Protein, quaternized proteins such as Hydroxypropyltrimonium Hydrolyzed Soy Protein, and acylated proteins such as Sodium Cocoyl Hydrolyzed Amaranth Protein. The conditioning agent may be present in an amount of from about 0 percent to about 5 percent, such as 0 percent to about 1 percent.

Examples of suitable chelating agents include Ethylenediamine tetraacetic acid (EDTA) and salts thereof, e.g. Tetrasodium EDTA; Tetrasodium Glutamate Diacetate; and Tetrasodium Iminodisuccinate. The chelating agent may be present in an amount of from about 0 percent to about 3 percent, such as from about 0 percent to about 1 percent.

Examples of suitable fillers include those which may also function as binders or to enhance the hardness or feel properties of the bar. Classes of suitable fillers include organic fillers such as Dextrin, Starch (e.g. Corn Starch, Mannitol, Wheat Flour) and inorganic fillers (e.g., Talc, Mica, aluminosilicate clays, Sodium Sulfate, carbonate salts, such as Calcium Carbonate, and phosphate salts such as Calcium Phosphate. Talc is a preferred filler. The filler may be present in an amount of from about 0 percent to about 60 percent.

Examples of suitable opacifying agents include colorants, including organic dyes, (e.g. Yellow 10 or Orange 4) and inorganic pigments (e.g. Iron Oxides or Ultramarines), in amounts suitable to produce visually appealing colors and/or optical effects. The opacifying agents may be present in an amount of from about 0 percent to about 2 percent, such as from about 0 to about 0.075 percent. Titanium dioxide is a preferred opacifying agent.

Examples of suitable exfoliants include polyethylene beads, synthetic wax beads, cellulose beads and fibers, jojoba ester beads, corn meal, walnut shell powder, and Luffa Cylindrica Fruit fiber. The exfoliants may be present in an amount of from about 0 percent to about 2 percent.

Examples of suitable preservatives include parabens, quaternary ammonium species, phenoxyethanol, benzoates, DMDM hydantoin. The preservatives may be present in an amount from about 0 to about 1 percent or from about 0.05 percent to about 0.5 percent.

Examples of suitable skin benefit agents include those suitable for use at pH less than about 8 and may include anti-aging agents, antimicrobial agents, anti-acne agents and the like. One suitable class of skin benefit agents are antimicrobial agents including organic acids and salts thereof, such as Alpha hydroxy acids, e.g. Glycolic Acid, Lactic Acid; Beta hydroxy acids, e.g. Salicylic Acid; and citric acid. The antimicrobial agents may be present in an amount from about 0 percent to about 4 percent, such as from about 0 to about 2 percent.

Cleansing bars of the present invention may further include superhydrophilic amphiphilic copolymers (SAC). Examples of suitable superhydrophilic amphiphilic copolymers are described in US 8,258,250.

In order to enhance mildness and other properties of the cleansing bar, the cleansing bars of the present invention have a pH of about 8 or less. According to certain embodiments the cleansing bar has a pH from about 3 to about 8, preferably from about 4 to about 7, more preferably from about 4 to about 6. To determine pH of the cleansing bar, a solution comprising 1% cleansing bar solids dissolved in deionized water is prepared. The pH of the solution is then determined by the ASTM method E70 - 07 Standard Test Method for pH of Aqueous Solutions with the Glass Electrode.

Cleansing bars of the present invention provide high foaming, particularly in comparison to comparable cleansing bars that do not include a PGE_{≤40}. According to certain embodiments, when tested according to the Cleansing Bar Foam Test detailed in this specification, cleansing bars of the present invention have a Maximum Foam Volume that is at least about 10% higher than their comparable cleansing bar without a PGE_{≤40}. According to certain other embodiments, cleansing bars of the present invention have a Maximum Foam Volume that is at least about 40% higher than their comparable cleansing bar without a PGE_{≤40}, preferably at least about 41 % higher, more preferably at least about 45% higher, even more preferably at least about 50% higher, even more preferably at least about 55% higher, and even more preferably at least about 100% higher than their comparable cleansing bar without a PGE_{≤40}. As one skilled in the art would readily understand and as defined herein the "comparable cleansing bar without a PGE_{≤40}" for any cleansing bar of the present invention means a cleansing bar with the same ingredients as the subject cleansing bar except with the PGE_{≤40} removed (i.e. a cleansing bar that has 0% by weight of PGE_{≤40} and wherein the additional material ("q.s.") to compensate for the omission of PGE_{≤40} is composed of equal proportions by weight of the other (non-PGE_{≤40}) ingredients in the cleansing bar). For example, see cleansing bar Inventive Examples E1 and E2 and its comparable cleansing bar without a PGE_{≤40}, Comparative Example C1, as described below.

Cleansing bars containing a PGE with a peak melting point greater than about 40 °C, Comparative Examples C2 and C3, show no increase or even a decrease in foam volume compared to the comparable cleansing bar without PGE, Comparative Example C1, as described below.

Cleansing bars of the present invention may be made by any of various methods. According to certain embodiments, an aqueous surfactant mixture is prepared by combining the hydrophobic binder, the non-soap anionic surfactant, the water soluble bar hardener , and water such that the mixture is rendered fluid, thereby permitting homogeneous mixing of the components. While the relative proportions of the hydrophobic binder, the non-soap anionic surfactant, the water soluble bar hardener, and water may be varied, typically substantially the entire formula amount of each of the hydrophobic binder and the non-soap anionic surfactant that are intended to be used in the final cleansing bar are used to prepare the aqueous surfactant mixture. According to certain embodiments only a portion of the total formula amount of and/or the water soluble bar hardener is used in preparation of the aqueous surfactant mixture. According to certain embodiments the aqueous surfactant mixture includes at least about 80%, preferably at least about 90% of combined non-soap anionic surfactant and hydrophobic binder, with the remainder consisting essentially of water soluble bar hardener and water. The amount of water in the aqueous surfactant mixture may be from about 0.25 percent to about 20 percent, preferably from about 0.5 percent to about 15 percent, more preferably from about 1 percent to about 15 percent, even more preferably from about 2 percent to about 15 percent, and even more preferably from about 3 percent to about 15 percent.

According to certain embodiments, in order to maintain a pH of about 8 or less, the amount of soap added during the process of making the bar is less than about 10 percent on a weight basis immediately prior to forming the cleansing bar, and is preferably less than about 5 percent, more preferably less than about 2.5 percent, even more preferably less than about 1 percent, such as less than 0.1 percent soap. While the inventors recognize that it is possible that a certain amount of soap may form in situ, according to certain embodiments, the heated aqueous surfactant blend includes less than about 10 percent of soap, preferably less than about 5 percent soap, more preferably less than about 1 percent soap immediately prior to cooling, and, in certain embodiments, is free of soap.

Prior to forming the final solid cleansing bar, the cleansing bar formulation blend may be subject to additional conventional processing steps. For example, according to one embodiment, the blend is flaked such as by contacting the blend with a metal roller which has been chilled such as by circulating cold water within the roller. The resulting material may comprise discrete, flaky structures. This flaked blend may then be mixed or "amalgamated," such as at ambient temperature. This mixing may be performed just before, during, or just after certain additional additives are mixed into the flaked blend.

According to certain other embodiments, the (flaked) blend is extruded (e.g., through an opening) to form an extruded surfactant mass. A cleansing bar is then formed by, for example, conventional processes such as cutting (e.g., with a blade) and/or stamping the extruded surfactant mass with a die to form the final bar shape. Whereas polymers used in conventional cleansing bars to allegedly improve performance tend to create mushy bars that are difficult to process with extrusion and/or stamping, the inventors have found that cleansing bars of the present invention and/or using the inventive processes are easily processed.

In certain embodiments, the compositions produced via the present invention are preferably used as or in personal care products for treating or cleansing at least a portion of the human body. Examples of certain preferred personal care products include various products suitable for application to the skin, hair, and/or vaginal region of the body, such as shampoos, hand, face, and/or body washes, bath additives, gels, lotions, creams, and the like. As discussed above, applicants have discovered unexpectedly that the instant methods provide personal care products having one or more of desirable properties such as foaming characteristics, reduced irritation, and/or improved manufacturability.

The present invention provides methods of treating and/or cleansing the human body comprising contacting at least a portion of the body with a composition of the present invention. Certain preferred methods comprising contacting mammalian skin, hair and/or vaginal region with a composition of the present invention to cleanse such region and/or treat such region for any of a variety of conditions including, but not limited to, acne, wrinkles, dermatitis, dryness, muscle pain, itch, and the like. In certain preferred embodiments, the contacting step comprises applying a composition of the present invention to human skin, hair or vaginal region.

The cleansing methods of the present invention may further comprise any of a variety of additional, optional steps associated conventionally with cleansing the skin including, for example, lathering, rinsing steps, and the like.

### EXAMPLES

The following tests are used in the instant methods and in the following Examples.

**Cleansing Bar Foam Test:** Determination of foam generated by the cleansing bar is measured in accordance with the following Cleansing Bar Foam Test. Pellets of bar-form products are used to determine their foam generating properties upon dissolution and agitation according to the present invention. The Cleansing Bar Foam test is conducted as follows: a pellet is fabricated by compressing shavings of a cleansing bar form product into a cylindrical pellet shape mold at 5000 psi (approx. weight of each pellet is 0.65 grams). To determine the Maximum Foam Volume, a solution of hard water (100 ppm Ca²⁺) is prepared by dissolving calcium chloride into deionized water and added to the sample tank of a SITA R-2000 foam tester (commercially available from Future Digital Scientific, Co.; Bethpage, N.Y.). The test parameters are set to repeat three runs (series count = 3) of 250 ml sample size (fill volume = 250 ml) with thirteen stir cycles (stir count = 17) for a 15 second stir time per cycle (stir time = 15 seconds) with the rotor spinning at 1200 RPM (revolution = 1200) at a temperature setting of 35° C ± 2° C. After the initial cycle where only the Ca²⁺ solution is stirred, the pellet is added to the sample tank (i.e. at time = 15 s). Foam volume data is collected at the end of each stir cycle (2-17) and the average and standard deviation of the three runs are determined. The Maximum Foam Volume is reported for each example as the value after the 17th stir cycle (255 seconds).

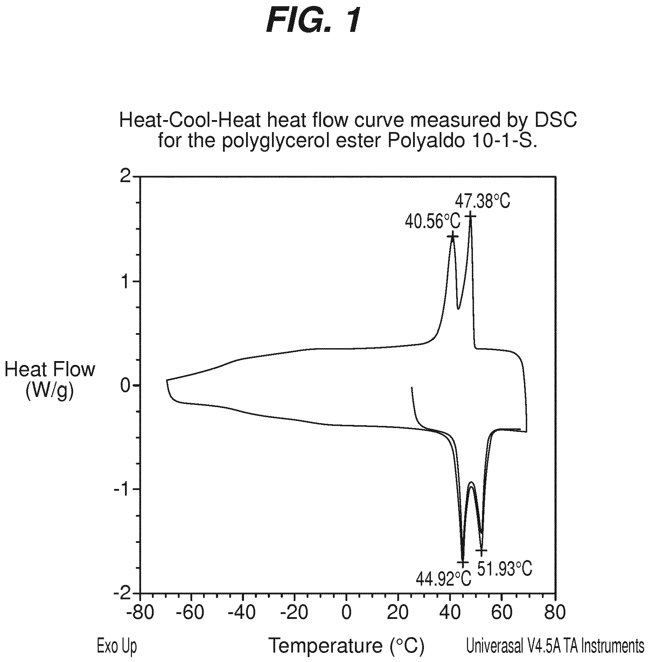

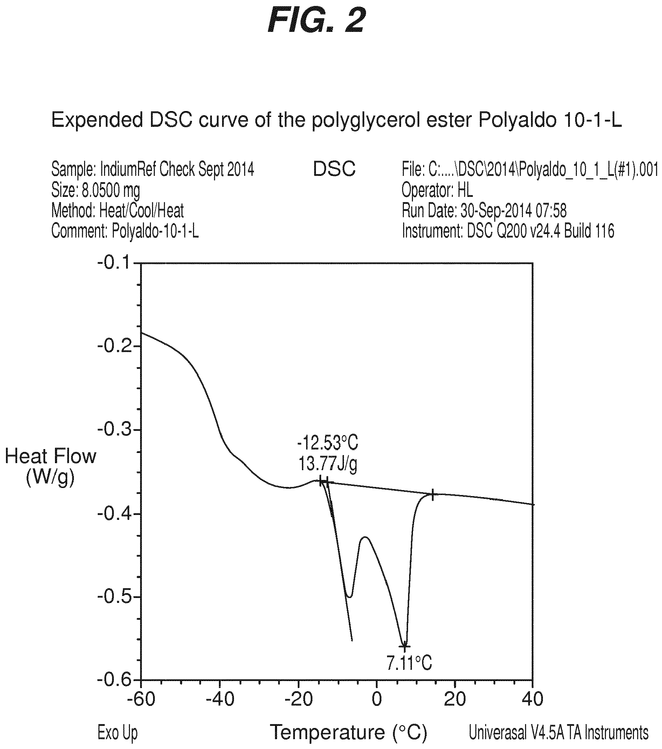

**Peak Melting Point Test using Differential Scanning Calorimetry (DSC):** Determination of the peak melting point of PGE is measured in accordance with the following DSC test. 6-8 mg PGE raw material samples were weighed and used directly for DSC measurement. The instrument used is a DSC-Q200: Thermal Analysis, from TA Instruments, Inc. A Heat-Cool-Heat program was used at 10°C/min for the cycles between -80°C to 70°C with a start temperature of 25°C. The measured heat flow vs temperature curves allow determination of phase transitions, their temperatures and enthalpies through peak-position and peak-area analysis. An example of a measured heat flow curve is shown in Figure 1. Transitions from solid to liquid states (melting) upon heating result typically in peaks pointing downward in the heat flow curves (it is convention that heat flow to the sample is negative and from the sample positive). Peak melting point temperature is determined by analyzing the peak position. The peak melting temperature is the temperature for which the heat flow peak has a minimum relative to the peak baseline. An example is shown in Figure 2.

**Infinite Rate Viscosity Test:** Determinations of infinite rate viscosity of the PGEs as applicable were conducted on a controlled-stress rheometer (AR-2000™, TA Instruments Ltd., New Castle, DE, USA). Steady-state shear rate sweeps were performed at 25.0 ±0.1 °C using a cone-plate geometry (20mm diameter, 2°) applying rates from 0.1 to 10 rad/s. Data acquisition and analysis were performed with the Rheology Advantage software v4.1.10 (TA Instruments Ltd., New Castle, DE, USA). Infinite rate viscosities were calculated via the fitting of viscosity vs shear rate data to a viscosity cross model. Except otherwise stated, viscosities are given in centiPoise (cps).

**Preparation of Cleansing Bars:** The cleansing bars of Examples E1, E2, and C1 through C3 were prepared according to the following procedure. Prior to bar preparation, the syndet base mixture (solid flakes comprised of sodium cocoyl isethionate, stearic acid, water, cocamidohydroxypropyl sultaine, sodium isethionate, sodium chloride, and titanium dioxide) was crushed using a mixer (Model 2000S-188Z, Lanqing Light Industrial Machinery Co., Ltd.) to yield a waxy powder. To an appropriately sized vessel were added crushed syndet base flake (1.90 kg) and the specified polyglycerol ester (0.10 kg). The contents of the vessel were mixed manually for two minutes using a spatula. The mixture was added to a lab-scale plodder (Mazzoni Soap Production Line Model SX-M-75) with cooling water set to keep the mixture at ambient temperature and refined twice. After refining twice, the mixture was transferred to a three-roll mill (Mazzoni Model RM-100/3) with cooling water set to keep the mixture at ambient temperature and milled once. Following milling, the mixture was returned to the plodder and was extruded at a temperature of approximately 40°C (e.g. 35°C to 40°C), to form a smooth, uniform billet. The billet was subsequently cut and die-stamped into bars using a soap stamping press (Mazzoni Model STA/N).

The compositions are shown in Table 1a and 1b.

**TABLE 1a**

| | | **Comparative Example C1** | **Comparative Example C2** | **Comparative Example C3** |
|---|---|---|---|---|
| **Trade Name** | **INCI Name** | **Formula Active (wt%)** | **Formula Active (wt%)** | **Formula Active (wt%)** |
| Syndet Base | Sodium Cocoyl Isethionate | 46.71 | 44.37 | 44.37 |
| | Stearic acid | 37.31 | 35.44 | 35.44 |
| | Water | 10.42 | 9.90 | 9.90 |
| | Cocamidopropyl Hydroxysultaine | 2.50 | 2.38 | 2.38 |
| | Sodium Isethionate | 1.99 | 1.89 | 1.89 |
| | Sodium Chloride | 0.54 | 0.51 | 0.51 |
| | Titanium Dioxide | 0.54 | 0.51 | 0.51 |
| Polyaldo 10-1-O | Polyglyceryl-10 Oleate | | | |
| Polyaldo 10-1-L | Polyglyceryl-10 Laurate | | | |
| Polyaldo HGDS-KFG | Polyglyceryl 6 Distearate | | | 5.00 |
| Polyaldo 10-1-S | Polyglyceryl-10 Stearate | | 5.00 | |
| **TOTAL** | | **100.00** | **100.00** | **100.00** |

**TABLE 1b**

| | | **Inventive Example E1** | **Inventive Example E2** |
|---|---|---|---|
| **Trade Name** | **INCI Name** | **Formula Active (wt%)** | **Formula Active (wt%)** |
| Syndet Base | Sodium Cocoyl Isethionate | 44.37 | 44.37 |
| | Stearic acid | 35.44 | 35.44 |
| | Water | 9.90 | 9.90 |
| | Cocamidopropyl Hydroxysultaine | 2.38 | 2.38 |
| | Sodium Isethionate | 1.89 | 1.89 |
| | Sodium Chloride | 0.51 | 0.51 |
| | Titanium Dioxide | 0.51 | 0.51 |
| Polyaldo 10-1-O | Polyglyceryl-10 Oleate | | 5.00 |
| Polyaldo 10-1-L | Polyglyceryl-10 Laurate | 5.00 | |
| Polyaldo HGDS-KFG | Polyglyceryl-6 Distearate | | |
| Polyaldo 10-1-S | Polyglyceryl-10 Stearate | | |
| **TOTAL** | | **100.00** | **100.00** |

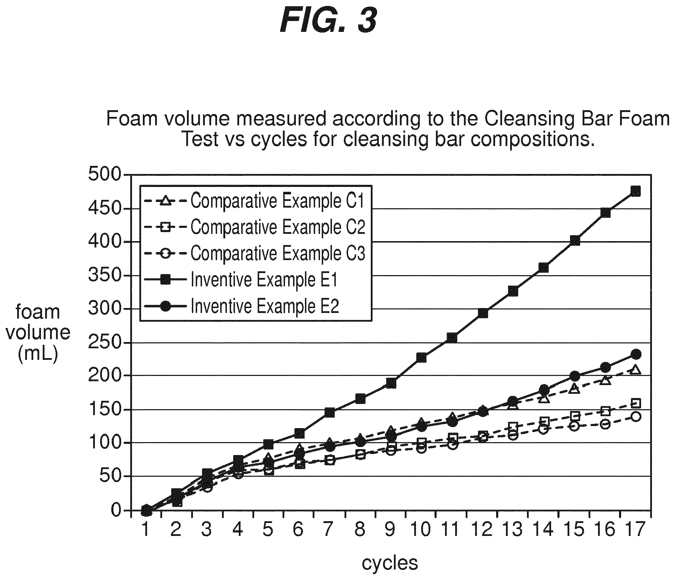

The foam volume data were generated using the Cleansing Bar Foam Test as described herein. The results are shown in Table 2 and Figure 3.

**Table 2: Cleansing Bar Foam Test: Foam Volume (mL) vs. Cycles for Inventive Examples E1 and E2 and Comparative Examples C1, C2 and C3.**

| | Comparative Example C1 | | Comparative Example C2 | | Comparative Example C3 | | Inventive Example E1 | | Inventive Example E2 | |
|---|---|---|---|---|---|---|---|---|---|---|
| Cycles | Average | Std Dev | Average | Std Dev | Average | Std Dev | Average | Std Dev | Average | Std Dev |
| 1 | 0 | 0 | 0 | 0 | 0 | 0 | 0 | 0 | 0 | 0 |
| 2 | 20 | 5 | 13 | 3 | 15 | 2 | 26 | 3 | 14 | 5 |
| 3 | 48 | 4 | 45 | 5 | 36 | 4 | 55 | 10 | 43 | 2 |
| 4 | 68 | 5 | 60 | 3 | 55 | 4 | 74 | 3 | 64 | 2 |
| 5 | 78 | 6 | 62 | 6 | 61 | 6 | 98 | 8 | 72 | 2 |
| 6 | 91 | 3 | 71 | 3 | 72 | 4 | 114 | 6 | 84 | 4 |
| 7 | 100 | 6 | 77 | 4 | 74 | 6 | 146 | 2 | 95 | 2 |
| 8 | 107 | 2 | 85 | 5 | 83 | 2 | 166 | 4 | 103 | 6 |
| 9 | 118 | 3 | 95 | 1 | 89 | 5 | 189 | 4 | 110 | 6 |
| 10 | 130 | 9 | 101 | 2 | 94 | 2 | 228 | 8 | 124 | 4 |
| 11 | 138 | 12 | 108 | 3 | 99 | 6 | 258 | 5 | 132 | 4 |
| 12 | 150 | 13 | 111 | 6 | 109 | 3 | 293 | 9 | 148 | 5 |
| 13 | 159 | 12 | 125 | 5 | 113 | 3 | 327 | 12 | 163 | 8 |
| 14 | 169 | 18 | 133 | 2 | 121 | 4 | 362 | 8 | 179 | 5 |
| 15 | 183 | 15 | 142 | 4 | 126 | 5 | 402 | 6 | 199 | 11 |
| 16 | 195 | 21 | 149 | 1 | 129 | 4 | 443 | 5 | 213 | 12 |
| 17 | 211 | 15 | 160 | 4 | 140 | 6 | 476 | 5 | 233 | 8 |

Applicants have discovered that when the inventive cleansing bar is prepared with PGE_{≤40}, foam performance is increased. To illustrate the improvement of foam performance in the inventive cleansing bars, Table 2 shows Foam Volume (in milliliter, mL) vs. cycles for Inventive Examples E1 and E2 and Comparative Examples C1, C2, and C3 (and Figure 3 shows this same data in plot form). It is clear from the Figure that the inclusion of the PGE_{≤40} markedly improves foaming performance. For example the Maximum Foam Volume of Inventive Example E1 (476 mL) represents a 125% increase in volume over Comparative Example C1, 197% over C2, and 240% over C3. The Maximum Foam Volume of Inventive Example E2 (233 mL) represents a 10% increase in volume over Comparative Example C1, 45% over C2, and 66% over C3.

As examples, Table 3 shows peak melting points, physical form at 25°C and viscosity, if applicable, of four PGEs, two with peak melting points of about 40°C or lower and two with peak melting points above 40°C. As can be seen in Inventive Examples E1 and E2 and Comparative Examples C2 and C3, the two PGEs with peak melting point below 40°C increase foam volume, whereas the two PGEs with peak melting point above 40°C do not impact or even decrease foam volume of the cleansing bars when compared to the comparable bar without PGE, Comparative Example C1.

**TABLE 3: Peak melting points, physical form and viscosities of PGEs used in Inventive Examples E1 and E2 and in Comparative Examples C2 and C3,**

| Used in | Polyglycerol ester | DSC peak melting point (°C) | Form at 25 °C | Viscosity (cPs) |
|---|---|---|---|---|
| Example E1 | Polyaldo 10-1-L | 7.11 | Liquid | 115k |
| Example E2 | Polyaldo 10-1-O | -28.63 | Liquid | 78k |
| Example C2 | Polyaldo 10-1-S | 44.63 | Solid (powder) | n/a |
| Example C3 | Polyaldo HGDS-KFG | 53.45 | Solid (pastilles) | n/a |

A non-exhaustive list of aspects of the invention is provided in the following numbered clauses:
1. A cleansing bar, comprising:
   a non-soap anionic surfactant;
   a polyglycerol ester having a peak melting point of about 40°C or less;
   a hydrophobic binder; and
   a water-soluble bar hardener, wherein the cleansing bar is characterized as having a pH of about 8 or less.
2. The cleansing bar of clause 1, wherein the polyglycerol moiety of the polyglycerol ester having a peak melting point of about 40°C or less has from about 2 to 20 repeating units.
3. The cleansing bar of clause 1, wherein the polyglycerol moiety of the polyglycerol ester having a peak melting point of about 40°C or less has from about 2 to 12 repeating units.
4. The cleansing bar of clause 1, wherein the polyglycerol moiety of the polyglycerol ester having a peak melting point of about 40°C or less has from about 4 to 10 repeating units.
5. The cleansing bar of clause 1, wherein the polyglycerol ester having a peak melting point of about 40°C or less is used in a concentration from greater than about 0.1% to about 25% by weight in the cleansing bar composition.
6. The cleansing bar of clause 1, wherein the polyglycerol ester having a peak melting point of about 40°C or less is used in a concentration from greater than about 0.1 % to about 10% by weight in the cleansing bar composition.
7. The cleansing bar of clause 1, wherein the polyglycerol ester having a peak melting point of about 40°C or less is used in a concentration from greater than about 0.1% to about 7% by weight in the cleansing bar composition.
8. The cleansing bar of clause 1, wherein the polyglycerol ester having a peak melting point of about 40°C or less is used in a concentration from greater than about 0.1% to about 5% by weight in the cleansing bar composition.
9. The cleansing bar of clause 1, wherein the polyglycerol ester having a peak melting point of about 40°C or less is used in a concentration from greater than about 1% to about 7.5% by weight in the cleansing bar composition.
10. The cleansing bar of clause 1, wherein the polyglycerol ester having a peak melting point of about 40°C or less is used in a concentration from greater than about 2% to about 6% by weight in the cleansing bar composition.
11. The cleansing bar of clause 1, wherein the polyglycerol ester having a peak melting point of about 40°C or less has from about 2 to 3 hydrophobic moieties.
12. The cleansing bar of clause 1 having a pH from about 4 to about 7.
13. The cleansing bar of clause 1, further comprising a zwitterionic surfactant.
14. The cleansing bar of clause 1, wherein the non-soap anionic surfactant is present in a concentration by weight of from about 30 percent to about 70 percent.
15. The cleansing bar of clause 1, wherein the non-soap anionic surfactant is selected from the group consisting of acyl isethionate, alkyl sulfosuccinate, α-sulfo fatty acid esters, α -sulfo fatty acids, alkyl glyceryl ether sulfonates and alkyl sulfates.
16. The cleansing bar of clause 1, wherein the hydrophobic binder is present in a concentration by weight of from about 5 percent to about 50 percent.
17. The cleansing bar of clause 1, wherein the hydrophobic binder is selected from the group consisting of fatty acids, fatty alcohols, esters of alcohols with fatty acids, polyol esters, waxes, mixed glycerides, triglycerides, hydrogenated tri glycerides and hydrogenated metathesis products of unsaturated triglycerides.
18. The cleansing bar of clause 1, wherein the water-soluble bar hardener is present in a concentration by weight of from about 0.25 percent to about 10 percent.
19. The cleansing bar of clause 1, wherein the water-soluble bar hardener is selected from inorganic metal cation salts of organic or inorganic acids.
20. The cleansing bar of clause 1, having a Maximum Foam Volume that is at least about 10% higher than its comparable cleansing bar without the PGE≤40, as measured by the Cleansing Bar Foam Test.
21. A cleansing bar, comprising:
   from about 30 percent to about 70 percent by weight of a non-soap anionic surfactant;
   a polyglycerol ester having a peak melting point of about 40 °C or less;
   from about 5 percent to about 50 percent by weight of a hydrophobic binder;
   from about 0.25 percent to about 10 percent by weight of a water-soluble bar hardener; and
   from about 0.1 to about 10 percent by weight of a zwitterionic surfactant, wherein the cleansing bar is characterized as having a pH of about 8 or less.

## Claims

1. A cleansing bar, comprising:
a non-soap anionic surfactant;
a polyglycerol ester having a peak melting point of about 40°C or less;
a hydrophobic binder; and
a water-soluble bar hardener, wherein the cleansing bar is characterized as having a pH of about 8 or less.

2. The cleansing bar of claim 1, wherein the polyglycerol moiety of the polyglycerol ester having a peak melting point of about 40°C or less has from about 2 to 20 repeating units; optionally
wherein the polyglycerol moiety of the polyglycerol ester having a peak melting point of about 40°C or less has from about 2 to 12 repeating units; optionally
wherein the polyglycerol moiety of the polyglycerol ester having a peak melting point of about 40°C or less has from about 4 to 10 repeating units.

3. The cleansing bar of claim 1 or claim 2, wherein the polyglycerol ester having a peak melting point of about 40°C or less is used in a concentration from greater than about 0.1% to about 25% by weight in the cleansing bar composition; optionally
wherein the polyglycerol ester having a peak melting point of about 40°C or less is used in a concentration from greater than about 0.1 % to about 10% by weight in the cleansing bar composition; optionally
wherein the polyglycerol ester having a peak melting point of about 40°C or less is used in a concentration from greater than about 0.1 % to about 7% by weight in the cleansing bar composition; optionally
wherein the polyglycerol ester having a peak melting point of about 40°C or less is used in a concentration from greater than about 0.1% to about 5% by weight in the cleansing bar composition.

4. The cleansing bar of claim 1 or claim 2, wherein the polyglycerol ester having a peak melting point of about 40°C or less is used in a concentration from greater than about 1% to about 7.5% by weight in the cleansing bar composition; optionally
wherein the polyglycerol ester having a peak melting point of about 40°C or less is used in a concentration from greater than about 2% to about 6% by weight in the cleansing bar composition.

5. The cleansing bar of any of the preceding claims, wherein the polyglycerol ester having a peak melting point of about 40°C or less has from about 2 to 3 hydrophobic moieties.

6. The cleansing bar of any of the preceding claims, wherein the polyglycerol ester having a peak melting point of about 40°C or less is selected from polyglyceryl-4 caprylate/caprate, polyglyceryl-5 caprylate/caprate, polyglyceryl-6 caprylate/caprate, polyglyceryl-7 caprylate/caprate, polyglyceryl-8 caprylate/caprate, polyglyceryl-9 caprylate/caprate, polyglyceryl-10 caprylate/caprate, polyglyceryl-4 caprate, polyglyceryl-5 caprate, polyglyceryl-6 caprate, polyglyceryl-7 caprate, polyglyceryl-8 caprate, polyglyceryl-9 caprate, polyglyceryl-10 caprate, polyglyceryl-4 laurate, polyglyceryl-5 laurate, polyglyceryl-6 laurate, polyglyceryl-7 laurate, polyglyceryl-8 laurate, polyglyceryl-9 laurate, polyglyceryl-10 laurate, polyglyceryl-6 cocoate, polyglyceryl-7 cocoate, polyglyceryl-8 cocoate, polyglyceryl-9 cocoate, polyglyceryl-10 cocoate, polyglyceryl-11 cocoate, polyglyceryl-12 cocoate, polyglyceryl-6 myristate, polyglyceryl-7 myristate, polyglyceryl-8 myristate, polyglyceryl-9 myristate, polyglyceryl-10 myristate, polyglyceryl-11 myristate, polyglyceryl-12 myristate, polyglyceryl-10 oleate, polyglyceryl-11 oleate, polyglyceryl-12 oleate, and combinations of two or more thereof; optionally
wherein the polyglycerol ester having a peak melting point of about 40 °C or less is selected from polyglyceryl-10 oleate, polyglyceryl-11 oleate, polyglyceryl-12 oleate, polyglyceryl-4 laurate, polyglyceryl-5 laurate, polyglyceryl-6 laurate, polyglyceryl-7 laurate, polyglyceryl-8 laurate, polyglyceryl-9 laurate, polyglyceryl-10 laurate and combinations of two or more thereof.

7. The cleansing bar of any of the preceding claims having a pH from about 4 to about 7.

8. The cleansing bar of any of the preceding claims, further comprising a zwitterionic surfactant.

9. The cleansing bar of claim 8, wherein the zwitterionic surfactant is selected from the group consisting of alkyl betaines, alkyl hydroxysultaines, alkylamidoalkyl betaines, alkylamidoalkyl hydroxysultaines, amphohydroxypropylsulfonates, and combinations of two or more thereof; optionally
wherein the zwitterionic surfactant is an alkylamidoalkyl hydroxysultaine; optionally
wherein the zwitterionic surfactant is cocamidopropyl hydroxysultaine.

10. The cleansing bar of any of the preceding claims, wherein the non-soap anionic surfactant is present in a concentration by weight of from about 30 percent to about 70 percent.

11. The cleansing bar of any of the preceding claims, wherein the non-soap anionic surfactant is selected from the group consisting of acyl isethionates, alkyl sulfosuccinates, α-sulfo fatty acid esters, α-sulfo fatty acid salts, alkyl sulfoacetates, alkyl sulfates, alkyl glyceryl ether sulfonates, alkoxyl hydroxypropyl sulfonates, α-olefin sulfonates prepared by sulfonation of long chain alpha olefins, alkyl sulfonates, paraffin sulfonates, alkylaryl sulfonates, linear alkyl benzene sulfonates, alkyl ether sulfates, alkyl monoglyceride sulfates, alkyl ether carboxylates, alkyl ether sulfosuccinates, dialkyl sulfosuccinates, alkylamidoalkyl sulfosuccinates, acyl glutamates, acyl aspartates, acyl taurates, acyl lactylates, acyl glycinates, acyl sarcosinates, anionic derivatives of alkyl polyglucosides and combinations of two or more thereof; optionally
wherein the non-soap anionic surfactant is selected from the group consisting of acyl isethionates, alkyl sulfosuccinates, α-sulfo fatty acid esters, α -sulfo fatty acids, alkyl glyceryl ether sulfonates and alkyl sulfates; optionally
wherein the non-soap anionic surfactant is an acyl isethionate; optionally wherein the non-soap anionic surfactant is sodium cocoyl isethionate.

12. The cleansing bar of any of the preceding claims, wherein the hydrophobic binder is present in a concentration by weight of from about 5 percent to about 50 percent.

13. The cleansing bar of any of the preceding claims, wherein the hydrophobic binder is selected from the group consisting of fatty acids, fatty alcohols, esters of alcohols with fatty acids, polyol esters, waxes, mixed glycerides, triglycerides, hydrogenated tri glycerides and hydrogenated metathesis products of unsaturated triglycerides; optionally
wherein the hydrophobic binder is selected from stearic acid or stearic alcohol and combinations thereof.

14. The cleansing bar of any of the preceding claims, wherein the water-soluble bar hardener is present in a concentration by weight of from about 0.25 percent to about 10 percent.

15. The cleansing bar of any of the preceding claims, wherein the water-soluble bar hardener is selected from inorganic metal cation salts of organic or inorganic acids; optionally
wherein the water-soluble bar hardener is an inorganic metal cation salt of isethionic acid, lactic acid, citric acid, hydrochloric acid or sulfuric acid and combinations of two or more thereof; optionally
wherein the water-soluble hardener is selected from sodium chloride and sodium isethionate and combinations thereof

16. The cleansing bar of any of the preceding claims, having a Maximum Foam Volume that is at least about 10% higher than its comparable cleansing bar without the PGE≤40, as measured by the Cleansing Bar Foam Test.

17. A cleansing bar of any of the preceding claims, comprising:
from about 30 percent to about 70 percent by weight of a non-soap anionic surfactant;
a polyglycerol ester having a peak melting point of about 40 °C or less;
from about 5 percent to about 50 percent by weight of a hydrophobic binder;
from about 0.25 percent to about 10 percent by weight of a water-soluble bar hardener; and
from about 0.1 to about 10 percent by weight of a zwitterionic surfactant, wherein the cleansing bar is characterized as having a pH of about 8 or less.
